# EUROPEAN PATENT APPLICATION

(11) **EP 4 070 847 A1**
(43) Date of publication of application: **12.10.2022**
(21) Application number: 22167279.3
(22) Date of filing: 08.04.2022
(51) Int. Cl.: A61M 37/00, A61M 5/32, A61B 17/34, A61D 7/00

(54) **SUBCUTANEOUS IMPLANT DELIVERY NEEDLE AND METHOD FOR DELIVERING AN IMPLANT SUBCUTANEOUSLY**

(30) Priority: 08.04.2021 NL 2027944
(71) Applicant: Animeasure B.V., 7683 RC Den Ham (NL)
(72) Inventor: Dijkstra, Freerk, 7552 AE Hengelo (NL); Koster, Willem, 7683 PE Den Ham (NL)
(74) Representative: Arnold & Siedsma

(57) **Abstract**

The application relates to a subcutaneous implant delivery needle (1), comprising a hollow shaft (2) formed by a circumferential wall (3), the shaft comprising a tip (4), wherein the circumferential wall has an open cross section at the tip. The tip comprises at its free end (5) a first longitudinal portion (6) which has at least one cutting edge (8,9), and a second longitudinal portion (7) removed from the free end, which has at least one blunt surface (11) substantially facing the free end. The application also relates to a method of delivering an implant subcutaneously, for instance using such a needle.

## Description

The invention relates to a subcutaneous implant delivery needle. In general, such needles are used to puncture the cutis (skin) and to deliver an implant subcutaneously (under the skin). Subcutaneous implants are used in both animals and humans. As an example, in animals chips may be introduced subcutaneously in order to permanently store information relating to their identity and/or owner. As an example in humans, hormones may be delivered using a contraceptive chip implanted under the skin.

In general, subcutaneous implant delivery needles comprise a hollow shaft formed by a circumferential wall, the shaft comprising a tip. The circumferential wall has an open cross section at the tip. After puncturing the cutis, the implant to be delivered is forced out through the needle, out the open cross section at the tip of the needle. Then, the needle is retracted.

The known needles have the disadvantage that they leave a relatively large trauma after delivering the implant. This is attributed to the fact that the needle punctures the skin, and thus leaves a trauma approximately the size of the outer dimensions of the needle. Especially when relatively large implants are to be delivered, which thus require relatively large needle sizes, this becomes a problem. In fact, the trauma will be correspondingly large. The larger the trauma, the more time recovery takes, and the larger the risk of infection.

As such, it is an object of the invention to decrease recovery time and/or to lower the risk of infection.

Said object is achieved by a subcutaneous implant delivery needle according to the preamble of claim 1, characterized in that the tip comprises at its free end a first longitudinal portion which has at least one cutting edge, and a second longitudinal portion removed from the free end, which has at least one blunt surface substantially facing the free end.

The cutting edge is used to create an incision in the cutis when the needle is inserted. As such, the cutis is cut rather than punctured. After creating the incision, the at least one blunt surface may push aside the cut skin, in order to allow introduction of a larger part of the needle. As a result, the needle may be inserted without having to cut a portion of the cutis that has similar dimensions as the needle. In fact, a relatively small incision may be pushed open by the blunt surface, after which the needle may be introduced. After removal of the needle, only the relatively small incision remains. The natural elasticity of the skin allows bringing together two sides of the skin on either side of the incision, so that the resulting trauma may heal easily and is less likely to be infected.

The cutting edge and the blunt surface may lie substantially in line with each other on the needle, so that the two acts of incising and pushing aside the cutis can be achieved in a single linear motion of the needle.

A cutting edge is herein defined as an edge sharp enough to cut the cutis, for instance upon the application of manual force. A blunt surface is herein defined as a surface more blunt than the cutting edge, in particular as blunt as to push skin aside rather than to cut it.

A clear distinction must be made between cutting and puncturing. In general, needles are used to puncture the skin. When puncturing, the entire surface area to puncture is contacted substantially simultaneously, and a relatively large force is applied to protrude the cutis to be punctured. When cutting however, a point of contact of the cutting instrument moves along a path to be incised, extending the incision as it goes. In the current disclosure, cutting rather than puncturing is achieved by providing a cutting edge that extends not only transversal to the needle, but also parallel. As such, when moving the needle in the longitudinal direction through the cutis, a point of contact with the cutting edge moves in the transversal direction. As such, a cutting action is created by simply moving the needle in the longitudinal direction thereof.

In an embodiment of the needle, the circumferential wall comprises a recess at the tip. The recess may reduce the size of the foremost part of the tip, thereby making it easier to incise the skin, in particular to create the beginning of an incision.

The length of the tip may be defined by the depth of the recess.

In another embodiment of the needle, the at least one cutting edge and the at least one blunt surface are both positioned on a rim defining the recess. The rim may be a rim of the circumferential wall.

Positioning the cutting edge on the rim may allow using a shape of the recess to define a shape of the cutting edge, in order to provide the transversal and longitudinal components thereof, so as to generate a cutting action when moving the needle longitudinally. Positioning the blunt surface on the rim allows creating a needle with a relatively small footprint, which may therefore be relatively easy to insert. Moreover, such an embodiment may be relatively easy to manufacture.

The at least one cutting edge may extend over between 40% and 80% of a circumference of the rim as seen in planar cross section of the needle, more preferably between 50% and 70%, and most preferably around 55%. The rest of the circumference the rim may be formed by the at least one blunt surface.

A cutting edge of this size has been shown to create a relatively small trauma, while still allowing insertion of the needle. It is envisioned to use a smaller cutting edge for subjects with a relatively flexible skin. Nevertheless, in practical applications the cutting edge may be of the above given size.

In case the needle is circular, the length of the cutting edge as seen in planar cross section of the needle, may also be defined as a part of a circumference of the needle. In particular, the cutting edge may extend over between 180 and 240 degrees, which substantially correspond to the 50% and 70% mentioned above. For application on e.g. calves, it has been found that a cutting edge spanning approximately 200 degrees is particularly suitable.

It is particularly advantageous if, as seen in cross section of the needle, the at least one cutting edge extends across substantially the entire width of the needle.

Accordingly, the cutis need only be deformed in a single direction for letting the needle in. This may reduce trauma and facilitate introduction of the needle.

The first portion may comprise two cutting edges which share an apex and extend away from each other in a direction away from the apex.

The cutting edges may meet at the apex in a sharp angle, thereby create a point. This may facilitate cutting an incision starting at the point and moving outwards with the two cutting edges as the needle is introduced.

Further, the two cutting edges starting at a sharp point, may create an arced, u-shaped or v-shaped incision. Such an incision may define in the cutis a flap that can hinge inwards by pressure thereagainst of the blunt surface. Accordingly, the flap forms a valve-like structure that allows insertion and removal of the needle. More importantly, the flap remains attached to the rest of the cutis at a portion of its circumference, so that it is continuously provided with blood. As such, after the needle has been removed, the flap can be replaced in its original position. The resulting trauma is therefore only the incision, which can heal relatively easily due to the presence of the flap.

The apex may be positioned at or near the outside of the circumferential wall. Accordingly, the cutting edges may run along an edge of the circumferential wall, thereby creating an incision also extending partly circumferentially, which is a practical way of providing the above-defined effect of cutting a flap.

It is particularly advantageous if the two cutting edges extend at mutually different angles with respect to a longitudinal direction of the needle, i.e. its axis. Accordingly, the needle tip will be asymmetric. The asymmetric shape may aid in focusing a cutting force more strongly onto one of the two edges. Accordingly, cutting may require a smaller total force.

In particular, a part of a first cutting edge extends substantially parallel to the longitudinal axis of the needle. The other, second cutting edge may extend at an angle to the longitudinal direction. This allows focusing a force applied to the needle to the second cutting edge, which may facilitate cutting. Accordingly, it is not strictly necessary that the first cutting edge is in fact used to cut. As such, the first cutting edge may be replaced, at least along the parallel part, by a blunt section. The entire first cutting edge, possibly replaced by a blunt edge, may extend substantially parallel to the longitudinal axis of the needle, while the other extends at an angle to the longitudinal direction/axis.

The resulting needle would have a single cutting edge extending from the apex to a side of the needle. The other cutting edge may extend over a shorter (e.g. zero) distance towards a straight portion, such as a blunt portion in the first longitudinal portion of the tip, or directly towards an intermediate edge.

The at least one blunt surface may transition smoothly into the circumferential wall. Such a configuration allows relatively easy introduction of the needle, by avoiding protrusions or the like that could block the needle from entering the body.

In particular, the at least one blunt surface is flat, or curved in maximally one dimension. If the blunt surface is flat or curved in only one dimension, it can exert a relatively large force on the cutis without causing damage to it. This is cause by a relatively large contact area posed by the surface.

It is possible to provide two separate blunt surfaces. Two blunt surfaces may be used to present multiple points of pressure which may push aside the cutis in order to allow the needle to enter. In particular, the blunt surfaces may be arranged as an extension of the cutting edges. It is also possible to provide a single blunt surface that extends along the rim from one cutting edge all the way to the other cutting edge. This particular configuration allows a relatively large contact area for pushing the cutis aside, thereby reducing the risk of damage. In such a case, the single blunt surface may be curved in multiple dimensions, as long as it remains blunt.

The tip may further comprise at least one intermediate edge between the at least one cutting edge and the at least one blunt surface, the intermediate edge extending along a portion of a length of the hollow shaft.

In particular, the intermediate edge may extend along the rim. Additionally or alternatively, the intermediate edge may extend parallel to the longitudinal direction of the needle.

During insertion of the needle, the intermediate edge may stop a cutting action of the cutting edge, before the blunt surface contacts the uncut cutis. Accordingly, the intermediate edge aids in limiting the length of the incision to the length of the cutting edge.

In one embodiment of the needle, the at least one cutting edge, the at least one blunt surface and the at least one intermediate edge together extend along the entire length of the rim. As such, the rim defines the entire front section of the needle. Such a needle may be manufactured relatively easily by machining the recess into the circumferential wall and thereby creating a rim of the desired shape.

It is particularly advantageous if the at least one cutting edge transitions smoothly into the at least one blunt surface. Such a smooth transition may facilitate introduction of the needle.

It is noted that the transition may include any intermediate edges.

The invention also relates to a pre-loaded subcutaneous implant delivery needle. The needle may be pre-loaded with a subcutaneous implant. Pre-loading the needle may remove the need for the end-user of the needle to insert the implant into the needle. As such, the implant may be made and kept sterile while it is in the needle, thereby reducing the likelihood of causing infections.

In order to facilitate delivering implants, the hollow shaft may be substantially cylindrical in shape, wherein optionally an outer diameter of the hollow shaft is between 8 and 16 mm, preferably between 10 and 14 mm, most preferably around 12 mm.

The invention also relates to a method of delivering an implant subcutaneously, the method comprising the following steps to be performed in any suitable order or at least partly simultaneously:
a) providing the implant in a needle as described herein;
b) incising the cutis with a cutting edge of the needle, thereby creating a pivotable cutis portion;
c) inserting the needle into the body up to and including at least the second longitudinal portion, thereby pushing inwards the pivotable cutis portion;
d) moving the implant out of the needle towards the tip thereof; and
e) removing the needle from the body.

The method may be used to deliver an implant subcutaneously with a relatively small amount of trauma. In particular, an arc, v-, or u-shaped incision may be made by the needle, which thus forms a pivotable cutis portion. Said portion corresponds to the above-described flap, which acts as a valve. After removal of the needle, the flap may move back to its original place, thereby allowing the incision to heal and reducing a risk of infection.

The invention will be further elucidated with reference to the drawings, in which;
Figure 1 shows schematically a perspective view of a first embodiment of a subcutaneous implant delivery needle;
Figures 2A and 2B show schematically a perspective view and a detail in top view of another embodiment of such a needle;
Figures 3A - 3C show schematically how the needle of figures 2A - 2B interacts with a portion of cutis; and
Figure 4 shows schematically the resulting trauma.

In the figures, like elements are referred to using like numerals. Across different embodiments, like elements are referred to using reference numerals increased with 100 (one hundred).

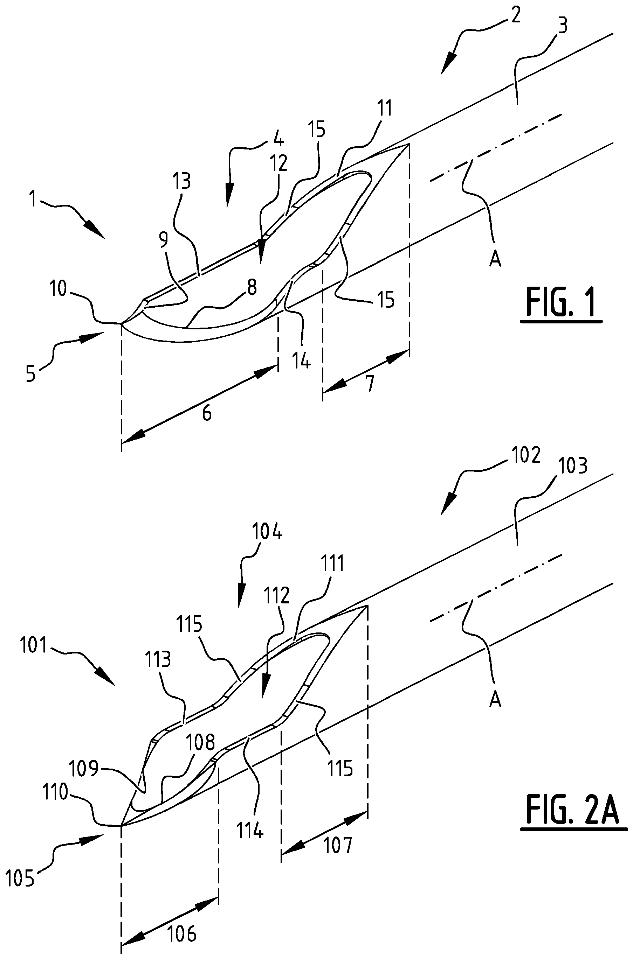

Figure 1 shows a subcutaneous implant delivery needle 1. The needle 1 comprises a hollow shaft 2 with an outer diameter of 12 mm, which is formed by a circumferential wall 3. The shaft 2 comprises a tip 4, which is open towards its free end 5. The tip 4 is divided in a first longitudinal portion 6 and a second longitudinal portion 7. The first portion 6 starts at the free end 5. The second longitudinal portion 7 is at a distance of the free end 5. The first portion 6 comprises two cutting edges 8, 9. The cutting edges 8, 9 meet at an apex 10. From the apex 10, both edges 8, 9 extend sideways at mutually different angles with respect to the longitudinal axis A of the needle 1.

The apex 10 lies at an outside of the circumferential wall 3. A first cutting edge 8 extends at a relatively small angle, whereas the second cutting edge 9 extends at a relatively large angle. The second portion 7 includes a blunt surface 11 that runs along a rim of a recess 12 and interconnects the two cutting edges. The blunt surface 11 faces the free end 5 of the needle 1. The blunt surface 11 has two substantially flat sections 15. As such, the blunt surface 11 forms a stop, that will engage the cutis when the needle protrudes into the cutis, and push the cutis aside in order to make room for the needle 1.

In between the cutting edges 8, 9 and the blunt surface 11 is are intermediate edges 13, 14, also part of the aforementioned rim, which extend substantially parallel to the longitudinal axis A. Together with the cutting edges 8, 9 and the blunt surface 11, the intermediate edges 13, 14 extend across the entire rim, and thus define the recess 12. The cutting edges 8, 9 together extend across approximately half of the rim. As seen in a planar cross section of the needle, the edges 8, 9 together cover approximately 180 degrees of a circle defined by the wall in that plane. As such, the cutting edges 8, 9 extend across the entire width of the needle 1. The cutting edges 8, 9 transition smoothly into the intermediate edges 13, 14, and into the blunt surface 11.

The shown needle 1 can be pre-loaded with a subcutaneous implant (not shown) i.e. be provided to an end user with the implant therein, in order to incise the cutis, an then deliver the cutis by pushing the implant out through the free end 5 of the needle 1.

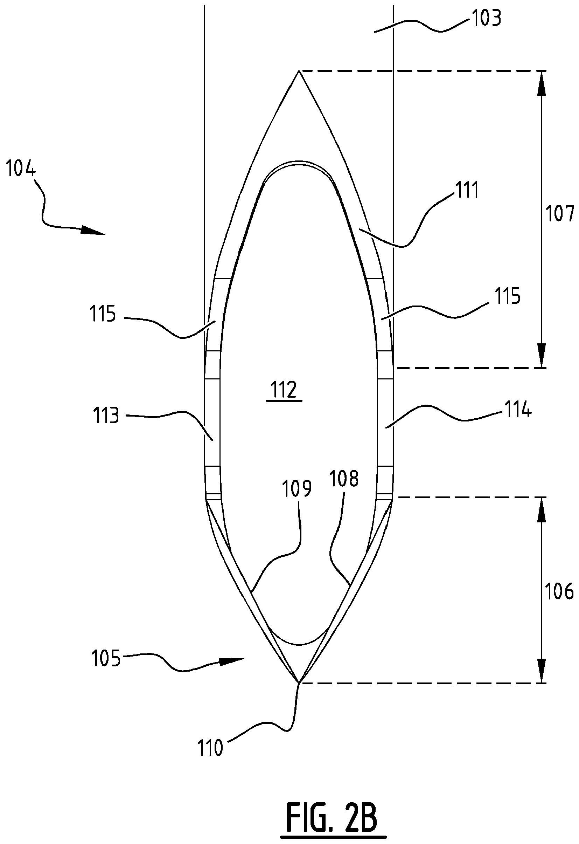

The needle 101 of figures 2A and 2B differs from the needle 1 of figure 1, in that the free edges 108, 109 extend at equal angles from the apex 10, so that the needle 101 has a generally symmetric shape.

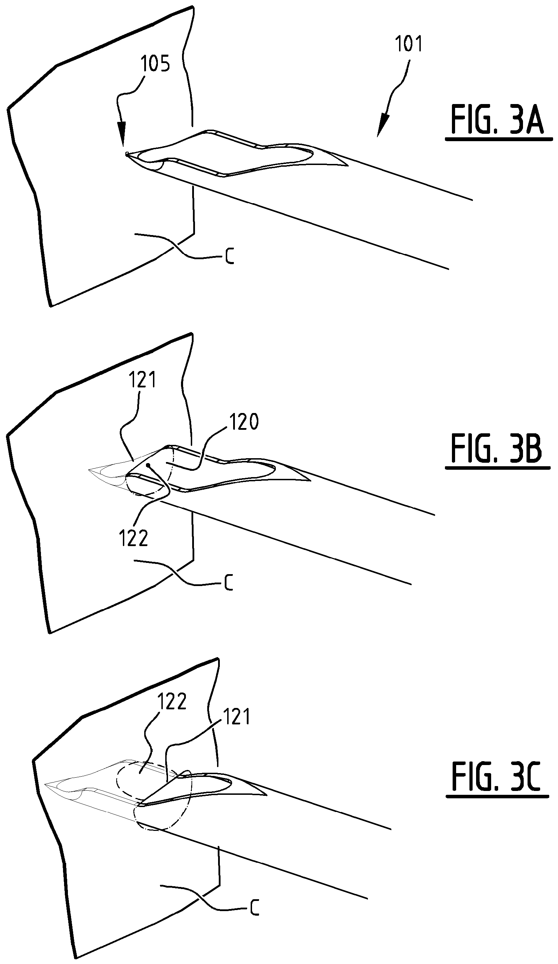

Figures 3A - 3C show how the needle 101 of figures 2A and 2B can be used to make an incision in the cutis C. Alternatively, the needle 1 of figure 1 could be used. First, the needle 101 is moved towards the cutis. The free end 105 is made to contact the cutis C, and then a force is applied, so that the cutting edges 108, 109 create an incision 120 in the cutis. The incision 120 is shown with a dashed-dotted line in figure 3B. In general, the incision 120 follows the shape of the cutting edges 108, 109 projected in the plane of the cutis C, which in the shown example is semi-circular. The incisions 120 defines a portion 122 of the cutis C that is pivotable with respect to the rest of the cutis along a line 121. Such a portion acts as a flap or valve, that can pivot open to allow the needle 101 to pass into the cutis C and close when the needle 101 has been removed. In figure 3C the flap 122 can be seen to pivot along line 121 into the body.

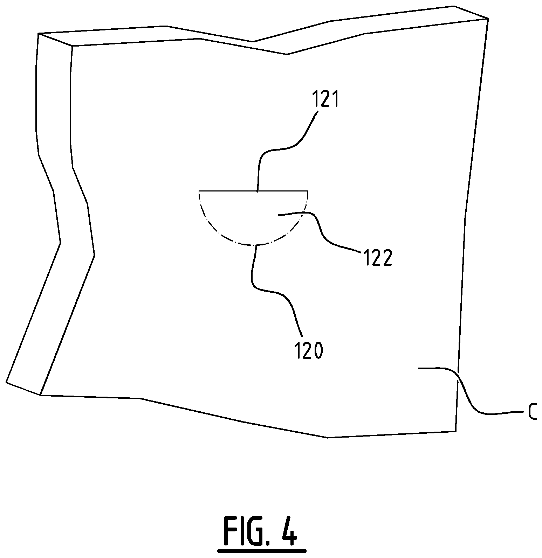

Figure 4 shows the cutis C of figures 3A - 3C in plan view, after the needle 101 has been removed. The incision 121 is shown to be semi-circular, and thereby defines a flap 122 that can pivot along a line 121. The flap 122 has been moved back to its original position, so that the resulting trauma to the skin is only the incision 120.

Although the invention has been described hereabove with reference to specific embodiments and examples, the invention is not limited thereto. In fact the scope of the invention is also covered by the claims, which now follow.

## Claims

1. Subcutaneous implant delivery needle, comprising a hollow shaft formed by a circumferential wall, the shaft comprising a tip, wherein the circumferential wall has an open cross section at the tip,
**characterized in that**
the tip comprises at its free end a first longitudinal portion which has at least one cutting edge, and a second longitudinal portion removed from the free end, which has at least one blunt surface substantially facing the free end.

2. Subcutaneous implant delivery needle according to the previous claim, wherein the circumferential wall comprises a recess at the tip.

3. Subcutaneous implant delivery needle according to the previous claim, wherein the at least one cutting edge and the at least one blunt surface are both positioned on a rim defining the recess.

4. Subcutaneous implant delivery needle according to any of the preceding claims, wherein the at least one cutting edge extends over between 40% and 80% of a circumference of the rim as seen in planar cross section of the needle, more preferably between 50% and 70%, and most preferably around 55%, and/or wherein, as seen in cross section of the needle, the at least one cutting edge extends across substantially the entire width of the needle.

5. Subcutaneous implant delivery needle according to any of the preceding claims, wherein the first portion comprises two cutting edges which share an apex and extend away from each other in a direction away from the apex.

6. Subcutaneous implant delivery needle according to the previous claim, wherein the apex is positioned at or near the outside of the circumferential wall.

7. Subcutaneous implant delivery needle according to any of claims 5 - 6, wherein the cutting edges extend at mutually different angles with respect to a longitudinal direction of the needle.

8. subcutaneous implant delivery needle according to at least claim 5 and claim 7, having a first cutting edge extending from the apex to a side of the needle, and another, possibly blunt, edge extending over a shorter distance towards a straight portion.

9. Subcutaneous implant delivery needle according to any of the preceding claims, wherein the at least one blunt surface smoothly transitions into the circumferential wall.

10. Subcutaneous implant delivery needle according to any of the preceding claims, wherein the at least one blunt surface is flat, or curved in maximally one dimension.

11. Subcutaneous implant delivery needle according to any of the preceding claims, comprising two separate blunt surfaces.

12. Subcutaneous implant delivery needle according to any of the preceding claims, wherein the tip further comprises at least one intermediate edge between the at least one cutting edge and the at least one blunt surface, the intermediate edge extending along a portion of a length of the hollow shaft.

13. Subcutaneous implant delivery needle according to any of claims 2 - 12, wherein the at least one cutting edge, the at least one blunt surface and the at least one intermediate edge together extend along the entire length of the rim.

14. Subcutaneous implant delivery needle according to any of the preceding claims, wherein:
- the at least one cutting edge transitions smoothly into the at least one blunt surface; and/or
- the needle is pre-loaded with a subcutaneous implant; and/or
- the hollow shaft is substantially cylindrical in shape, wherein optionally an outer diameter of the hollow shaft is between 8 and 16 mm, preferably between 10 and 14 mm, most preferably around 12 mm.

15. Method of delivering an implant subcutaneously, the method comprising the following steps to be performed in any suitable order or at least partly simultaneously:
a) providing the implant in a needle according to any of the preceding claims;
b) incising the cutis with a cutting edge of the needle, thereby creating a pivotable cutis portion;
c) inserting the needle into the body up to and including at least the second longitudinal portion, thereby pushing inwards the pivotable cutis portion;
d) moving the implant out of the needle towards the tip thereof; and
e) removing the needle from the body.
